# EUROPÄISCHE PATENTSCHRIFT

(11) **EP 2 758 783 B1**
(45) Veröffentlichungstag und Bekanntmachung des Hinweises auf die Patenterteilung: **08.09.2021**
(21) Anmeldenummer: 12762572.1
(22) Anmeldetag: 19.09.2012
(51) Int. Cl.: B01L 3/00, B01L 9/00

(54) **EIN IMMUNOBLOT VERFAHREN**
AN IMMUNOBLOT METHOD
UN PROCÉDÉ D'IMMUNOTRANSFERT

(30) Priorität: 24.09.2011 DE 202011106093 U
(43) Veröffentlichungstag der Anmeldung: 30.07.2014
(73) Patentinhaber: Euroimmun Medizinische Labordiagnostika AG, 23560 Lübeck (DE)
(72) Erfinder: MEYER, Wolfgang, 23689 Pansdorf (DE); SCHEPER, Thomas, 23919 Berkenthin (DE)
(86) Internationale Anmeldenummer: PCT/EP2012/068381
(87) Internationale Veröffentlichungsnummer: WO 2013/041540

(56) Entgegenhaltungen:
- DE-A1- 10 000 322
- US-B1- 6 406 862
- Organtec Diagnostika Gmbh: "ORG 710-08 / ORG 710-16 ANA-9-Line Membranfixierter Immunoblot für die semiquantitative Bestimmung von nukleären-Autoantikörpern", , 1. Januar 2011 (2011-01-01), Seiten 1-4, XP055045420, Gefunden im Internet: URL:http://www.orgentec.com/products/pdfs/ Immunoblot_de_IFU_ORG_710.pdf [gefunden am 2012-11-23] in der Anmeldung erwähnt
- VERTOSICK F T ET AL: "Antibodies to native and denatured DNA", JOURNAL OF IMMUNOLOGICAL METHODS, ELSEVIER SCIENCE PUBLISHERS B.V.,AMSTERDAM, NL, Bd. 102, Nr. 1, 24. August 1987 (1987-08-24), Seiten 15-21, XP026141897, ISSN: 0022-1759, DOI: 10.1016/S0022-1759(87)80004-2 [gefunden am 1987-08-24]
- None

## Beschreibung

Die Erfindung betrifft ein Verfahren zum Nachweis von Antikörpern in einer Patientenprobe mit wenigstens einer Inkubationsrinne, in die ein Immunoblot-Streifen einlegbar ist und dieser mit einer Patientenprobe, einem Konjugat und einem Substrat inkubierbar ist.

Auf dem Gebiet der medizinischen Labordiagnostik sind unterschiedliche Testsysteme bekannt, mit denen das Vorhandensein spezieller Antikörper in Patientenproben überprüft wird. Da Antikörper im Körper eines Patienten nach einer viralen oder bakteriellen Infektionskrankheit gebildet werden, können mit derartigen Tests akute oder aber bereits von dem Patienten durchgemachte Krankheiten diagnostiziert werden. Ebenso können bei Auswahl entsprechend geeigneter Antigene Autoimmunerkrankungen oder Allergien diagnostiziert werden.

Üblicherweise erfolgt der Nachweis von Antikörpern im Wege der sogenannten Stufendiagnostik, bei der zunächst ein empfindliches Screening und dann eine spezifische Bestätigung durchgeführt werden. In der Routineserologie werden hierbei für das Screening oftmals ELISAs (Enzyme-linked Immunosorbent Assay) verwendet, während als Bestätigungstest vornehmlich Immuno-Blot-Streifen, insbesondere Western-Blot-Streifen, Dot-Blot-Streifen oder Linienblot-Streifen, eingesetzt werden.

Durch den Nachweis spezieller Patientenantikörper und deren Zugehörigkeit zu bestimmten Immunglobulinklassen ist es möglich, sowohl Rückschlüsse auf eine bereits stattgefundene oder eine akut bestehende bakterielle oder virale Infektion als auch über den zeitlichen Verlauf einer Infektion zu machen. So handelt es sich bei Antikörpern der Immunglobulinklasse IgM um sogenannte Frühphasen-Antikörper, während Antikörper der Immunglobulinklasse IgG üblicherweise Spätphasen-Antikörper darstellen.

Um zu gewährleisten, dass die jeweiligen Tests korrekt durchgeführt worden sind und somit die richtigen Ergebnisse zeigen, sind Immunoblots mit sogenannten Funktions- oder Reaktionskontrollbanden bekannt, durch die entweder die Zugabe des Serums oder des Konjugats überprüft werden kann. Das Konjugat enthält hierbei einen Antikörper, der an den spezifischen Antikörper im Patientenserum gekoppelt wird und der in der Regel von einer anderen Tierspezies stammt. Die entsprechenden Kontrollbanden erscheinen immer dann, wenn ein Konjugat einer beliebigen Ig-Klasse zugefügt worden ist.

Mit diesen Testsystemen ist es demgegenüber allerdings nicht möglich, Sicherheit darüber zu erlangen, ob das für den Test jeweils benötigte Konjugat einer speziellen Ig-Klasse verwendet wurde. So würde bei einem derart gestalteten Test eine Bande immer dann erscheinen, wenn der Teststreifen mit einem Konjugat inkubiert wurde und zwar unabhängig davon, ob IgM-, IgG- oder IgA-Konjugat verwendet wurde. Auf diese Weise könnte beispielsweise eine Borrelienerkrankung in der Frühphase übersehen werden, sofern ein IgM-Teststreifen fälschlicherweise mit IgG-Konjugat inkubiert wurde, da die Kontrollbande zwar erschiene, der eigentliche Test aber ein negatives Ergebnis liefere. Somit würde in diesem Fall eine Therapie in der Frühphase der Erkrankung unterbleiben und die Infektion könnte unbemerkt in die Spätphase wechseln.

Aus diesem Grund ist es wünschenswert, wenn anhand eines Teststreifens erkannt werden könnte, ob die Zugabe des Serums erfolgt ist und ob die Funktionalität des zugegeben Konjugats korrekt ist.

In diesem Zusammenhang ist aus der DE 100 00 322 A1 ein Immunoblot-Streifen mit wenigstens zwei unterschiedlichen Kontrollzonen bekannt. Die beschriebenen Teststreifen verfügen einerseits über eine Serumkontrollzone, die die erfolgte Inkubation des Teststreifens mit Patientenserum durch eine entsprechende Kontrollbande anzeigt, sowie andererseits über wenigstens eine Konjugatkontrollzone, die die erfolgte Inkubation des Teststreifens mit einem markierten Anti-Patienten-Immunglobulin-Antikörper aus einer anderen Tierspezies anzeigt. Durch das Vorsehen mehrerer unterschiedlicher Konjugatkontrollzonen kann mit einem entsprechenden Teststreifen bestimmt werden, ob das für den Test vorgesehene Konjugat tatsächlich verwendet worden ist. Weiterhin ist optional vorgesehen, auf dem Teststreifen eine Cut-Off-Sensorzone vorzusehen, mittels der die Einhaltung der korrekten Inkubationszeit überprüfbar ist.

Als Alternative zur Cut-Off-Sensorzone auf dem Teststreifen ist es ferner bekannt, einen separaten vorentwickelten Kalibrationsstreifen vorzusehen, um die Farbintensität der Cut-Off-Kontrolle auf dem Patientenstreifen mit der entsprechenden Sensorzone des Kalibrationsstreifens zu vergleichen (s. Gebrauchsanweisung ORG 710 ANA-9-Line [710-0803-02-d] der Orgentec Diagnostika GmbH, März 2008). Die Cut-Off-Kontrolle des Patientenstreifens wird an die entsprechende Bande auf dem Kalibrationsstreifen "angelegt" und die Farbintensitäten werden verglichen. Auf diese Weise soll überprüft werden, ob der Test unter zulässigen Bedingungen durchgeführt worden ist.

Aus der US 6406862 B1 ist ein Verfahren zur Detektion einer Konzentration eines C-reaktiven Proteins bekannt, bei welchem Signale, die proportional zu einer Menge des eines C-reaktiven Proteins aus einer Probe sind, mit einem Standard verglichen werden.

Aus ,Vertosick F.T. et.al., "Antibodies to native and denatured DNA", Journal of Immunological Methods, Elsevier Science Publishers B.V., Amsterdam, NL, Vol. 102, No. 1', ist ein Verfahren bekannt, bei welchem vorgeschlagen wird, Kalibrationsstreifen zusammen mit Blotstreifen mit dem gleichen Serum Sample zu Inkubieren und anhand bekannter Mengen von IgG auf den einzelnen Kalibrationsstreifenabschnitten und den erfassten Intensitäten der Kalibrationsstreifenabschnitte als auch den Intensitäten aus den Slot-Slots darauf schließen zu können, wie viel Material an nDNA auf den Slot-Blots vorhanden ist.

Problematisch an den aus dem Stand der Technik bekannten Lösungen ist, dass oftmals die tatsächlich im Labor herrschenden Versuchsbedingungen bei der Auswertung der Tests nicht zufriedenstellend berücksichtigt werden. So lässt etwa ein bereits vorinkubierter Teststreifen keine Rückschlüsse darüber zu, in welcher Intensität die einzelnen Banden unter unterschiedlichen Laborbedingungen erscheinen würden. Ebenso gilt es als problematisch, dass anhand der vielfach verwendeten Positiv- und/oder Negativkontrollen zwar Aussagen darüber gemacht werden können, ob die Inkubation mit Patientenserum und/oder mit dem korrekten Konjugat erfolgt ist, dagegen sind quantitative Aussagen in Bezug auf die Testergebnisse, vor allem unter Berücksichtigung spezieller Eigenschaften der auf dem jeweiligen Teststreifen erscheinenden Banden, nicht möglich. Weiterhin überprüfen die bekannten Testsysteme entweder nicht die Einhaltung der korrekten Inkubationszeit unter Berücksichtigung der tatsächlich vorliegenden Labor- und Testbedingungen oder die entsprechende Auswertung ist mit erheblichem Aufwand verbunden, da jeder Teststreifen einzeln überprüft werden muss. In diesem Zusammenhang sind beispielsweise Testkits bekannt, bei denen eine Cut-Off-Zone auf jedem Testsreifen vorgesehen ist und eine Inkubation zu stoppen ist, sobald eine Cut-Off-Bande auf dem Teststreifen erscheint. Eine derartige Testdurchführung erscheint insbesondere im Hinblick auf die Entwicklung im Bereich der Labortechnik hin zu einem immer höheren Grad der Automatisierung, nachteilig, da ein Teststreifen während der gesamten Inkubation beobachtet werden muss.

Ausgehend von den bekannten Testsystemen sowie den hiermit verbundenen Problemen liegt der Erfindung die Aufgabe zugrunde, ein Testsystem zur Auswertung von Immunoblot-Streifen anzugeben, das mit verhältnismäßig einfachen Mitteln eine Berücksichtigung der tatsächlich herrschenden Versuchsbedingungen ermöglicht.

Die der Erfindung zugrunde liegende Aufgabe wird mit einem Verfahren nach Anspruch 1 gelöst. Vorteilhafte Ausführungsformen der Erfindung sind Gegenstand der abhängigen Ansprüche und werden in der folgenden Beschreibung unter teilweiser Bezugnahme auf die Figuren näher erläutert.

Nicht ein Teil der Erfindung ist eine Vorrichtung zum Nachweis von Antikörpern in einer Patientenprobe mit wenigstens einer Inkubationsrinne, in die ein Immunoblot-Streifen einlegbar ist und dieser mit einer Patientenprobe, einem Konjugat und einem Substrat inkubierbar ist sowie mit einem Kontrollmittel zur visuellen Überprüfung einer Qualität der Inkubation derart weitergebildet worden, dass als Kontrollmittel ein separater Kalibrationsstreifen mit wenigstens zwei Kontrollzonen vorgesehen ist, der in eine weitere Inkubationsrinne einlegbar und mit einer bekannten Referenzprobe, dem Konjugat und dem Substrat inkubierbar ist, so dass nach erfolgter Inkubation anhand eines Vergleichs einer Farbintensität der in den wenigstens zwei Kontrollzonen des Kalibrationsstreifens erscheinenden Kontrollbanden mit einer Farbintensität zumindest einer Bande des wenigstens einen inkubierten Inkubationsstreifen ein Gütewert für eine Antikörper-Erregerprotein-Reaktion ermittelbar ist. Unter einem Gütewert wird verstanden, dass im Hinblick auf wenigstens ein Kriterium die Qualität des durchgeführten Tests ermittelbar ist.

Mit der vorgeschlagenen technischen Lösung wird ein Testsystem, der nicht Teil der Erfindung ist, zur Verfügung gestellt, mit dem auf einfache Weise die Qualität einer Inkubation unter Berücksichtigung der während der Inkubation tatsächlich herrschenden Laborbedingungen überprüft werden kann. Hierbei ist es denkbar, die Intensität der auf dem Kalibrationsstreifen erscheinenden Kontrollbanden mit wenigstens einer auf einem Patientenstreifen erscheinenden Bande zu vergleichen und/oder unter Berücksichtigung der wenigstens zwei Kontrollbanden auf dem Kalibrationsstreifen eine Kalibrationskurve zu erstellen, die schließlich bei der Auswertung der Patientenstreifen berücksichtigt wird.

Dabei erscheinen die wenigstens zwei Kontrollbanden auf dem Kalibrationsstreifen bei gleicher Antikörperkonzentration in der Patientenprobe mit unterschiedlich starker Intensität. Die Intensitäten der Kontrollbanden werden erfasst und aus diesen Werten eine Kalibrationskurve erstellt. Auf der Grundlage dieser Kalibrationskurve ist es einerseits möglich, Aussagen über die Qualität der erfolgten Inkubation zu machen und andererseits die auf den Patientenstreifen erscheinenden Banden zumindest teilweise quantitativ auszuwerten. Um die Kalibration weiter zu verbessern, sind vorzugsweise mehr als zwei, insbesondere fünf Kontrollbanden auf dem Kalibrationsstreifen vorgesehen, um eine Kalibrationskurve zu erstellen. Sobald nach Abschluss eines Tests eine Kalibrationskurve erstellt worden ist, wird überprüft, ob die Kalibrationskurve innerhalb des auf dem Zertifikat der Charge, der der jeweilige Test angehört, angegebenen Wertebereichs liegt.

Es ist möglich, dass auf dem Kalibrationsstreifen eine Kontrollzone für eine Negativkontrolle vorgesehen ist. In der Kontrollzone der Negativkontrolle erscheint üblicherweise keine Bande, da diese entweder nur sehr dünn beschichtet ist oder auf Antikörper reagiert, die im Normalfall nicht in der Patientenprobe zu erwarten sind. Sofern in der Kontrollzone der Negativkontrolle eine Bande erscheint, sollte in jedem Fall die Ordnungsmäßigkeit des durchgeführten Tests überprüft werden, da es zumindest sehr unwahrscheinlich ist, dass die untersuchte Referenzprobe positiv in Bezug auf in dieser Kontrollzone befindliche Antigen reagiert.

Weiterhin ist auf dem Kalibrationsstreifen eine Kontrollzone für eine Positivkontrolle vorgesehen. In dieser Kontrollzone erscheint eine Bande, wenn der Kalibrationsstreifen mit der Patientenprobe inkubiert worden ist. Anhand der Positivkontrolle wird überprüft, ob der Kalibrationsstreifen und damit die im gleichen Testlauf inkubierten Patientenstreifen mit der Patientenprobe inkubiert worden sind.

Zudem ist es denkbar, eine Cut-Off-Kontrollzone auf dem Kalibrationsstreifen vorzusehen. Mit Hilfe der Cut-Off-Kontrolle wird überprüft, ob der für eine Inkubation der Teststreifen vorgesehene Zeitraum eingehalten worden ist. Wesentlich bei der Durchführung der Tests ist stets, dass der Kalibrationsstreifen unter den gleichen Bedingungen wie die gemeinsam mit diesem prozessierten Patientenstreifen inkubiert wird.

Möglich ist, dass auf einem ausgeführten Kalibrationsstreifen eine Kontrollzone vorgesehen ist, auf der eine Bande erscheint, sobald der Kalibrationsstreifen mit Konjugat inkubiert worden ist. In diesem Zusammenhang verfügt der Kalibrationsstreifen vorteilhafterweise über wenigstens zwei Konjugatkontrollzonen, die auf Antikörper der Patientenprobe unterschiedlicher Immunglobulinklassen reagieren. Bevorzugt sind wenigstens eine IgG- sowie eine IgM-Konjugatkontrolle vorgesehen. Eine ganz spezielle Weiterbildung sieht darüber hinaus vor, dass zusätzlich zu einer IgG- und einer IgM- auch eine IgA-Konjugatkontrollzone vorgesehen ist.

Mit den zuvor beschriebenen Konjugatkontrollzonen wird sicher gestellt, dass nicht nur überprüft wird, ob der Kalibrationsstreifen sowie der wenigstens eine gemeinsam mit diesem inkubierte Patientenstreifen mit einem Konjugat inkubiert worden ist, sondern es ist darüber hinaus auch überprüfbar, ob das jeweils richtige bzw. benötigte Konjugat verwendet worden ist.

Die Verwendung des Konjugats der vorgeschriebenen Immunglobulinklasse ist insofern von Bedeutung, da es in Abhängigkeit der jeweiligen Erkrankung zu einer Bildung von sogenannten Frühphasen-Antikörpern (IgM) und Spätphasen-Antikörpern (IgG) kommt. Eine Patientenprobe, die Frühphasen- aber keine Spätphasen-Antikörper, bspw. gegen Borrelien, enthält, zeigt normalerweise eine positive IgM- und eine negative IgG-Bande. Sollte irrtümlicherweise ein IgM-Patientenstreifen mit einem IgG-Konjugat inkubiert worden sein, könnte dies zu einem falschen Testergebnis führen, wobei insbesondere falsch negative Testergebnisse, etwa eine nicht erkannte Frühphaseninfektion, gravierende Folgen für den Patienten haben kann. Aus diesem Grund ist sowohl die Verwendung von Patientenstreifen als auch von Kalibrationsstreifen mit getrennten Zonen für die Detektion von Antikörpern unterschiedlicher Immunoglobulinklassen vorteilhaft.

In diesem Zusammenhang ist es ebenfalls denkbar, dass nicht oder zumindest nicht nur die Verwendung des korrekten Konjugats sondern die Anwesenheit der zu untersuchenden Antikörperklasse überprüft wird. Durch eine derart erweiterte Testdurchführung kann die Patientenprobe dahingehend untersucht werden, ob bei dem Patienten ein Antikörpermangelsyndrom vorliegt. Ein Testergebnis kann somit mit einem höheren Genauigkeitsgrad abgesichert werden.

Die Sichtbarmachung der Antigen-Antikörperbindung wird bevorzugt mittels markierter Antikörper gegen den Primärantikörper aus einer anderen Tierspezies durchgeführt. Vorzugsweise werden hierbei enzymkonjugierte Antikörper des Kaninchens oder der Ziege verwendet.

Alternativ ist es möglich, dass der Testkit derart ausgeführt ist, dass der Teststreifen nach der Inkubation mit der Patientenprobe nicht mit einem Sekundärantikörper einer anderen Tierspezies, sondern direkt mit einem anti-Human-IgG inkubiert und dann gewaschen wird.

Da das vorgeschlagene Testsystem nicht ein Teil der Erfindung ist und darauf beruht, dass die Patientenstreifen gemeinsam mit wenigstens einem Kalibrationsstreifen inkubiert werden, so dass die Inkubation der Streifen unter den gleichen Versuchsbedingungen erfolgt, ist es ausreichend, lediglich auf dem Kalibrationsstreifen geeignete Kontrollzonen für eine Positiv-, Negativ-, Konjugat- und/oder Cut-Off-Kontrolle vorzusehen und den Kalibrationsstreifen nach erfolgter Inkubation zu überprüfen. Auf die ansonsten oftmals auf den Patientenstreifen vorgesehenen einzelnen zusätzlichen Kontrollzonen kann verzichtet werden, so dass auf diesen mehr Platz für weitere Antigene vorhanden ist.

Im Folgenden wird das Verfahren sowie die dafür verwendeten Vorrichtungen, die alleine jedoch nicht Teil der Erfindung sind, ohne Beschränkung des allgemeinen Erfindungsgedankens anhand von Ausführungsbeispielen unter Bezugnahme auf die Figuren näher erläutert. Hierbei zeigen:
- Fig. 1:: herkömmlicher Western-Blot-Streifen,
- Fig. 2:: Inkubationswanne,
- Fig. 3:: Kalibrationsstreifen sowie
- Fig. 4:: Grafische Darstellung der Kalibrationskurve.

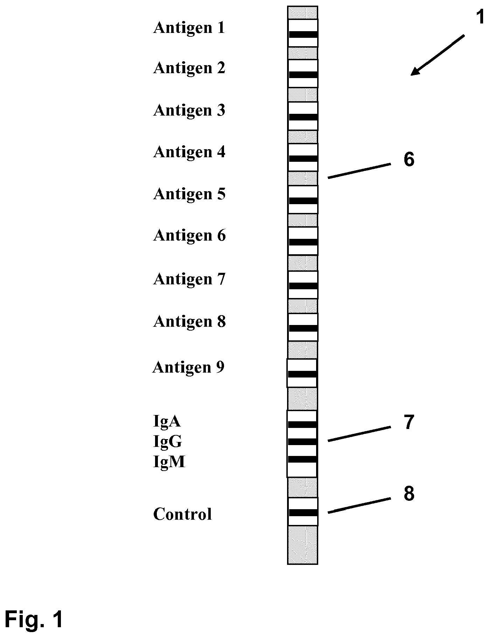

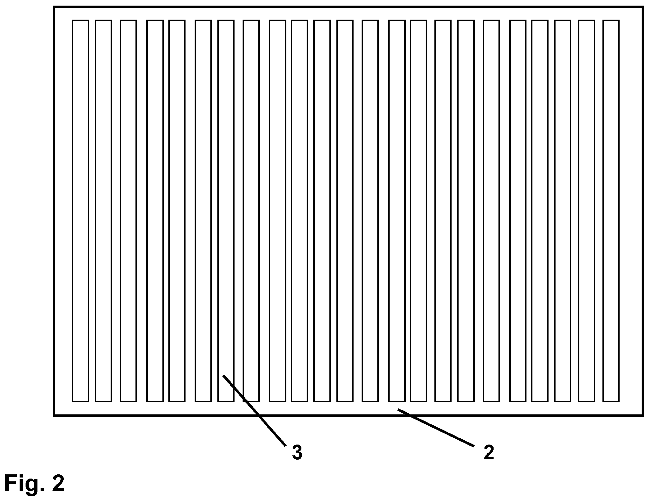

Figur 1 zeigt einen Western-Blot-Teststreifen 1 zur Untersuchung einer Patientenprobe auf eine mögliche Borrelieninfektion. Zur Untersuchung wird der Western-Blot-Streifen 1 zunächst in eine Inkubationsrinne 3 eingelegt und mit einem Patientenserum inkubiert. Für die Inkubation wird üblicherweise eine Inkubationswanne 2 verwendet, wie sie in Figur 2 gezeigt ist. Die Inkubationswanne 2 verfügt über eine Mehrzahl von Inkubationsrinnen 3, so dass eine entsprechende Anzahl von Teststreifen gleichzeitig inkubiert werden kann.

Die Inkubation des Teststreifens 1 oder der Teststreifen erfolgt mit einem enzymgekoppelten Konjugat sowie einem Substrat. Alternativ ist es denkbar, den Test derart zu gestalten, dass anstelle des Konjugats ein anti-Human-IgG verwendet wird. Im Folgenden wird die Testdurchführung lediglich beispielhaft unter Verwendung eines markierten Sekundärantikörpers einer anderen Tierspezies beschrieben, was daher keine Beschränkung des Erfindungsgegenstandes darstellen soll.

Sofern das Serum Antikörper aufgrund einer Borrelieninfektion aufweist, binden diese an die auf dem Teststreifen immobilisierten Erregerproteine. Mit Hilfe des Konjugats wird der gebundene Antikörper dann durch einen zweiten Antikörper einer anderen Tierspezies (Kaninchen, Ziege), der mit einem Enzym markiert ist, nachgewiesen. Dieser Nachweis erfolgt durch Zugabe des Substrats, das mit dem Enzym des Konjugats auf geeignete Weise reagiert. Das Testergebnis zeigt sich schließlich als Bandenmuster auf dem Western-Blot-Streifen, das das Vorhandensein einer Borrelieninfektion anzeigt. Hierfür sind auf dem in Figur 1 dargestellten Patientenstreifen 1 in der Testzone 6 neun verschiedene Antigene immobilisiert, die an Antikörper binden, welche spezifisch für eine Borrelieninfektion sind.

Die Durchführung des zuvor beschriebenen Tests, insbesondere die Abfolge der Inkubationsschritte, weist eine Vielzahl von möglichen Fehlerquellen auf. Daher besteht vor allem in der Routineserologie der Bedarf, die Qualität der einzelnen Inkubationsschritte zu überwachen. Aus diesem Grund sind auf dem in Figur 1 dargestellten Western-Blot-Streifen zusätzliche Kontrollzonen vorgesehen. Als Kontrollzonen sind eine Konjugatkontrollzone 7, in der sich eine Bande zeigt, sofern eine Inkubation mit einem Konjugat erfolgt ist, sowie eine Positivkontrolle 8, die eine Inkubation mit Patientenserum anzeigt, vorgesehen. Der Patientenstreifen 1 verfügt somit einerseits in der Testzone 6 über Bereiche, in denen bei Auftreten einer Erreger-Protein-Reaktion entsprechende Banden dargestellt werden. Andererseits sind Serum- und Konjugatkontrollzonen 7, 8 vorgesehen. Wesentlich hierbei ist, dass die Konjugatkontrollzone 7 wiederum in zwei ggf. drei unterschiedliche Bereiche unterteilt ist, in denen jeweils eine Bande erscheint sofern IgG- oder IgM-Konjugat bzw. bei Vorhandensein eines dritten Bereichs IgA-Konjugat inkubiert worden ist. Weitere Funktions- oder Reaktionskontrollbanden weist der in Figur 1 dargestellte Patientenstreifen 1 nicht auf.

Mit dem in Figur 1 dargestellten Teststreifen 1 wird auf herkömmliche Weise die Diagnose einer Borrelieninfektion durchgeführt, wobei üblicherweise eine Mehrzahl von Teststreifen 1 in eine Inkubationswanne 2, die eine Vielzahl paralleler Inkubationsrinnen 3 aufweist, eingelegt und inkubiert wird. Auf diese Weise können zeitgleich eine Vielzahl von Patientenstreifen 1 inkubiert und somit unterschiedliche Patientenseren auf eine mögliche Borrelieninfektion untersucht werden. Eine Inkubationswanne 2, oftmals auch als Tray bezeichnet, die über eine Mehrzahl von Inkubationsrinnen 3 verfügt, ist in Figur 2 dargestellt. Die Inkubationsrinnen 3 sind auf bevorzugte Weise derart ausgeführt, dass einerseits die Streifen 1 ausreichend Platz in den Inkubationsrinnen haben und gut von den für die Inkubation verwendeten Medien überströmt werden können sowie andererseits nicht unnötig viel Medium für die Inkubation verwendet werden muss.

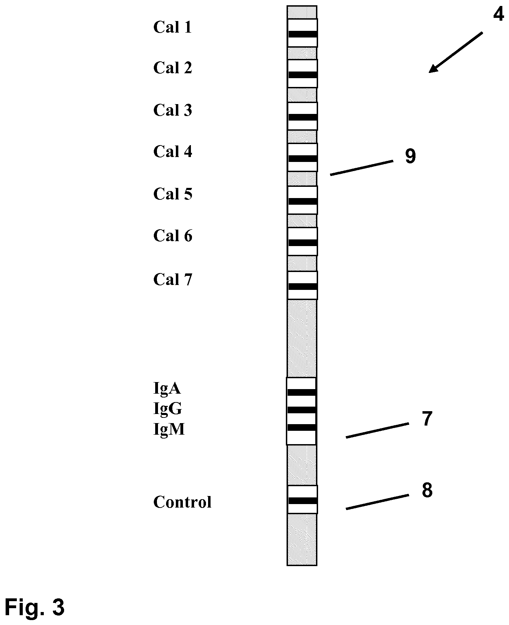

In Figur 3 ist ein Kalibrationsstreifen 4 dargestellt, wie er in einer Ausführungsform des erfindungsgemäß ausgeführten Verfahrens verwendet wird. Der Kalibrationsstreifen 4 verfügt über eine Kalibrierzone 9, eine Konjugatkontrollzone 7 sowie eine Positivkontrolle 8. Der Kalibrationsstreifen 4 ist als separater Streifen vorgesehen, der gemeinsam mit dem bzw. den Patientenstreifen 1, und somit unter gleichen Versuchsbedingungen, inkubiert wird. Zur Inkubation werden sowohl ein Kalibrationsstreifen 4 als auch die Patientenstreifen 1 in getrennte Inkubationsrinnen 3 einer Inkubationswanne 2 eingelegt, wobei die Patientenstreifen 1 mit Patientenserum, Konjugat und Substrat und der Kalibrationsstreifen 4 mit einer Referenzprobe, Konjugat und Substrat inkubiert werden.

Der Kalibrationsstreifen 4 verfügt in seiner Kalibrierzone über sieben Bereiche, in denen nach ordnungsgemäßer Inkubation mit der Referenzprobe unter den tatsächlich herrschenden Laborbedingungen jeweils Kalibrierbanden erscheinen, die sich in ihrer Intensität von einander unterscheiden.

Ferner ist eine Konjugatkontrollzone 7 mit drei verschiedenen Bereichen vorgesehen, wobei in diesen Bereichen jeweils in Abhängigkeit des verwendeten Konjugats, also je nachdem ob IgG-, IgM- oder IgA-Konjugat verwendet worden ist, eine Bande erscheint. Das Vorsehen einer Konjugatkontrolle 7 mit mehreren Bereichen ermöglicht somit nicht nur die Überprüfung, ob ein Konjugat inkubiert worden ist, sondern darüber hinaus auch, ob das Konjugat der jeweils benötigten Konjugatklasse verwendet worden ist.

Weiterhin verfügt der Kalibrationsstreifen 4 über eine Positivkontrollzone 8, in der eine Bande erscheint, sobald eine Inkubation des Streifens mit Patientenserum erfolgt ist.

In Ergänzung zur Positivkontrolle 8 ist es denkbar eine Negativkontrollzone vorzusehen, die so dünn mit einem Antigen beschichtet ist, dass unter normalen Bedingungen in dieser Zone keine Bande erscheint. Sofern sich nach erfolgter Inkubation eine Bande in diesem Bereich zeigt, sollte der Test in jedem Fall wiederholt werden, um eine Falschdiagnose mit Sicherheit auszuschließen.

Neben den zuvor beschriebenen Kontrollzonen ist es ferner denkbar auf dem Kalibrationsstreifen 4 eine Cut-Off-Kontrollzone vorzusehen, in der eine Cut-Off-Kontrollbande erscheint, sobald der Inkubationszeitraum ausreichend lang war, um zuverlässige Testergebnisse zu gewährleisten.

Der in Figur 3 dargestellte Kalibrationsstreifen 4 verfügt somit einerseits über eine in sieben Bereiche unterteilte Kalibrierzone 9, in der Banden unterschiedlicher Intensität entstehen, sobald eine Inkubation ordnungsgemäß durchgeführt worden ist, sowie andererseits über Bereiche für eine Konjugat- 7 und eine Positivkontrolle.

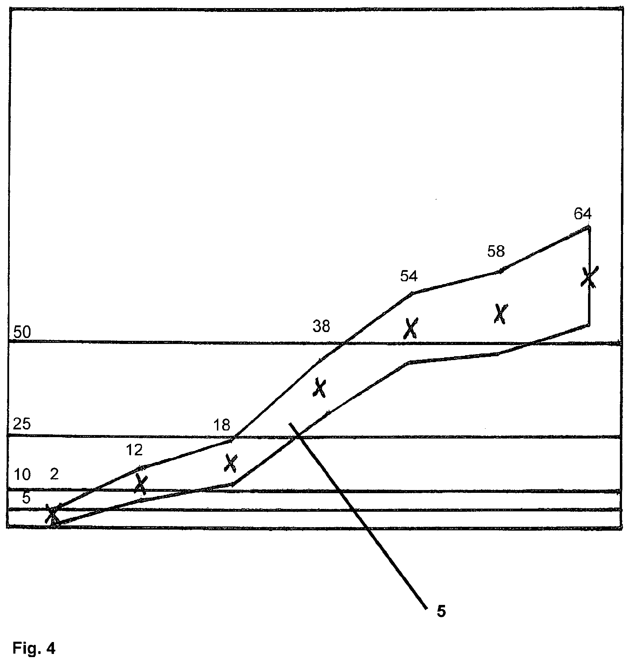

Wesentlich hierbei ist das Vorsehen einer Kalibrationszone 9, in der nach ordnungsgemäßer Inkubation mit einer bekannten Referenzprobe Kontrollbanden unterschiedlicher Intensität erscheinen. Auf der Grundlage dieser sieben Kontrollbanden wird schließlich eine Kalibrationskurve 5 erstellt, wie sie der Figur 4 zu entnehmen ist. Nach Erstellung der Kalibrationskurve 5 wird zunächst überprüft, ob sich die Kalibrationskurve innerhalb der für die entsprechende Charge eines Testkits zulässigen Grenzwerte befindet. Sofern dies der Fall ist, ist dies ein Beleg für eine ordnungsgemäße Testdurchführung. Es werden die auf dem oder den gleichzeitig mit dem Kalibrationsstreifen 4 inkubierten Patientenstreifen 1 in der Testzone 6 erschienenen Banden mit den Werten der Kalibrationskurve 5, also mit den Intensitäten der sieben Banden in der Kalibrationszone 9 auf dem Kalibrationsstreifen 4, verglichen.

Anhand eines Vergleichs der auf dem Kalibrationsstreifen 4 erschienenen Banden mit den entsprechenden Banden des Patientenstreifens 1 ist es möglich, die Qualität der durchgeführten Inkubation zu überwachen. Anhand des Vergleichs wird eine zumindest teilweise quantitative Auswertung des inkubierten Patientenstreifens 1 vorgenommen. Hierzu werden die in Bezug auf die Antigene 1 bis 7 auf dem Patientenstreifen 1 in der Testzone 6 dargestellten Banden mit den Banden in der Kalibrationszone 9 des Kalibrationsstreifens 4 verglichen. Auf der Grundlage eines Vergleichs der Intensitäten der Banden kann auf den jeweiligen Titer der untersuchten Patientenprobe geschlossen werden.

Bevorzugt finden sowohl die Erstellung der Kalibrationskurve 5 als auch die Erfassung der auf dem Patientenstreifen 1 erschienenen Banden und/oder der Vergleich der Patientenbanden mit den Kalibrationsbanden automatisch statt. Nach erfolgter Inkubation werden die Teststreifen 1 auf eine geeignete Unterlage aufgelegt oder - geklebt und die Oberflächen des Kalibrationsstreifens 4 und der Patientenstreifen 1 abfotografiert oder abgescannt und an eine elektronische Datenverarbeitungseinheit, auf der vorzugsweise eine spezielle Laborsoftware betrieben wird, übertragen.

In Ergänzung ist es denkbar, dass eine Auswertesoftware von dem die aufgenommene Farbintensität repräsentierenden Eingangssignal einen durch die Aufnahme des Hintergrundes bedingten Signalanteil abzieht.

Die zentrale Datenverarbeitungseinheit wertet die Intensitäten der einzelnen Banden aus, so dass die Kalibrationskurve 5 und die entsprechenden Testergebnisse über eine Ausgabeeinheit, vorzugsweise einen Monitor oder einen Drucker ausgegeben werden können.

Anhand eines Vergleichs der Intensitäten der sieben Kalibrationsbanden in der Kalibrationszone 9 des Kalibrationsstreifens 4, die unterschiedliche Intensitäten aufweisen, mit den auf dem oder den Patientenstreifen 1 in den Testzonen 6 gezeigten Banden wird eine Aussage über die Antikörperkonzentration in der untersuchten Patientenprobe erhalten und somit ein zumindest teilweise quantifiziertes Testergebnis erhalten. Wesentlich hierbei ist, dass die Inkubation des Kalibrationsstreifens 4 unter denselben Versuchsbedingungen wie die des oder der Patientenstreifen 1 stattgefunden hat.

### Bezugszeichenliste

- 1: Patientenstreifen
- 2: Inkubationswanne
- 3: Inkubationsrinne
- 4: Kalibrationsstreifen
- 5: Kalibrationskurve
- 6: Testzone
- 7: Konjugatkontrollzone
- 8: Positivkontrolle
- 9: Kalibrationszone

## Patentansprüche

1. Verfahren zum Nachweis von Antikörpern in einer Patientenprobe
wobei ein Kalibrationsstreifen (4) als separater Streifen gemeinsam mit einem Patientenstreifen (1) unter gleichen Versuchsbedingungen inkubiert wird,
wobei zur Inkubation sowohl der Kalibrationsstreifen (4) als auch der Patientenstreifen (1) in getrennte Inkubationsrinnen (3) einer Inkubationswanne (2) eingelegt werden,
wobei ferner der Patientenstreifen (1) mit Patientenserum, Konjugat und Substrat inkubiert wird und wobei ferner der Kalibrationsstreifen (4) mit einer Referenzprobe, Konjugat und Substrat inkubiert wird,
wobei der Kalibrationsstreifen (4) in einer Kalibrationszone (9) über sieben Bereiche verfügt, in denen nach ordnungsgemäßer Inkubation mit der Referenzprobe unter tatsächlich herrschenden Laborbedingungen jeweils Kalibrierbanden erscheinen, die sich in ihrer Intensität voneinander unterscheiden,
wobei ferner auf der Grundlage der sieben Kalibrierbanden eine Kalibrationskurve (5) erstellt wird,
wobei nach Erstellung der Kalibrationskurve (5) geprüft wird, ob sich die Kalibrationskurve (5) innerhalb von für die entsprechende Charge eines Testkits zulässigen Grenzwerten befindet
wobei die auf dem Patientenstreifen (1) erschienenen Banden mit den Werten der Kalibrationskurve (5) verglichen werden, und
wobei anhand des Vergleichs eine zumindest teilweise quantitative Auswertung des Patientenstreifens (1) vorgenommen wird.

2. Verfahren nach Anspruch 1,
wobei die Erstellung der Kalibrationskurve (5) als auch eine Erfassung von auf dem Patientenstreifen (1) erschienenen Banden automatisch stattfindet.

3. Verfahren nach Anspruch 2,
wobei nach erfolgter Inkubation die Teststreifen (1, 4) auf eine geeignete Unterlage aufgelegt oder geklebt werden und die Oberflächen des Kalibrationsstreifens (4) und des Patientenstreifens (1) abfotografiert oder abgescannt und an eine elektronische Datenverarbeitungseinheit, auf der vorzugsweise eine spezielle Laborsoftware betrieben wird, übertragen werden.

4. Verfahren nach Anspruch 3,
wobei eine Auswertesoftware von dem die aufgenommene Farbintensität repräsentierenden Eingangssignal einen durch die Aufnahme des Hintergrundes bedingten Signalanteil abzieht.

## Claims

1. Method for detecting antibodies in a patient sample,
wherein a calibration strip (4) as a separate strip is incubated together with a patient strip (1) under the same test conditions,
wherein both the calibration strip (4) and the patient strip (1) are placed into separate incubation channels (3) of an incubation trough (2) for the incubation,
wherein furthermore the patient strip (1) is incubated with patient serum, conjugate and substrate and wherein furthermore the calibration strip (4) is incubated with a reference sample, conjugate and substrate,
wherein the calibration strip (4) has, in a calibration zone (9), seven regions in which calibration bands respectively appear following proper incubation with the reference sample under actually prevailing laboratory conditions, which calibration bands differ from one another in terms of their intensity,
wherein furthermore a calibration curve (5) is created on the basis of the seven calibration bands,
wherein a check is carried out following creation of the calibration curve (5) to determine whether the calibration curve (5) is within limits permissible for the corresponding batch of a test kit,
wherein the bands appearing on the patient strip (1) are compared with the values of the calibration curve (5), and
wherein an at least semi-quantitative evaluation of the patient strip (1) is performed on the basis of the comparison.

2. Method according to Claim 1,
wherein the creation of the calibration curve (5) and capture of bands appearing on the patient strip (1) take place automatically.

3. Method according to Claim 2,
wherein the test strips (1, 4) are placed or adhered on a suitable base after incubation has been carried out and the surfaces of the calibration strip (4) and the patient strip (1) are photographed or scanned and transmitted to an electronic data processing unit on which specific laboratory software is preferably operated.

4. Method according to Claim 3,
wherein evaluation software subtracts from the input signal representing the recorded colour intensity a signal component due to the recording of the background.

## Revendications

1. Procédé de détection d'anticorps dans un échantillon de patient
une bande d'étalonnage (4) étant incubée en tant que bande séparée conjointement avec une bande de patient (1) dans les mêmes conditions de test,
pour effectuer l'incubation la bande d'étalonnage (4) et la bande de patient (1) étant placées toutes les deux dans des canaux d'incubation séparés (3) d'une cuve d'incubation (2),
en outre la bande de patient (1) étant incubée avec du sérum de patient, un conjugué et un substrat et en outre la bande d'étalonnage (4) étant incubée avec un échantillon de référence, un conjugué et un substrat,
la bande d'étalonnage (4) disposant dans une zone d'étalonnage (9) de sept zones dans lesquelles des bandes d'étalonnage, qui diffèrent les unes des autres par leur intensité, apparaissent après une incubation correcte avec l'échantillon de référence dans des conditions de laboratoire réelles,
une courbe d'étalonnage (5) étant en outre créée sur la base des sept bandes d'étalonnage,
après la création de la courbe d'étalonnage (5), une vérification étant effectuée pour savoir si la courbe d'étalonnage (5) se situe dans les valeurs limites autorisées pour le lot correspondant d'un kit de test
les bandes qui apparaissent sur la bande de patient (1) étant comparées aux valeurs de la courbe d'étalonnage (5), et
une évaluation au moins partiellement quantitative de la bande de patient (1) étant effectuée sur la base de la comparaison.

2. Procédé selon la revendication 1,
la création de la courbe d'étalonnage (5) et la détection des bandes qui apparaissent sur la bande de patient (1) étant effectuées automatiquement.

3. Procédé selon la revendication 2,
après incubation, les bandes de test (1, 4) étant placées ou collées sur un support approprié et les surfaces de la bande d'étalonnage (4) et de la bande de patient (1) étant photographiées ou scannées et transmises à une unité de traitement électronique de données sur laquelle un logiciel de laboratoire spécial est de préférence exécuté.

4. Procédé selon la revendication 3,
un logiciel d'évaluation soustrayant une composante de signal, due à l'enregistrement de l'arrière-plan, du signal d'entrée représentant l'intensité de couleur enregistrée.
